# EUROPEAN PATENT APPLICATION

(11) **EP 2 615 068 A1**
(43) Date of publication of application: **17.07.2013**
(21) Application number: 12194794.9
(22) Date of filing: 05.01.2012
(51) Int. Cl.: C02F 11/18, C10L 5/44, A61L 2/07

(54) **Apparatus and method for continuous preparation of disintegrated biomass sludge**

(62) Divisional of application: 12150269.4
(71) Applicant: CSL Carbon Solutions Ltd, Helier Jersey JE 4 9WG10 (GB)
(72) Inventor: Stark, Dr. Arne, 12623 Berlin (DE); Maas, Dr. Robert, 14471 Potsdam (DE)
(74) Representative: HOFFMANN EITLE

(57) **Abstract**

The invention relates to an apparatus for the continuous preparation of a disintegrated biomass sludge, the apparatus comprising a first positive displacement pump (2), a first piping (6, 12) connecting an outlet of the first positive displacement pump (2) to an inlet of the first positive displacement pump (2) so as to form a closed loop, a first disintegration device (20) which is provided in the first piping (6, 12) and configured to disintegrate a biomass sludge supplied thereto, a second pump (14) and a third pump (16). An outlet of the second pump (14) is connected to the first piping (6, 12) and an inlet of the third pump (16) is connected to the first piping (6, 12) upstream of a position where the outlet of the second pump (14) is connected to the first piping (6, 12).

## Description

### Field of the Invention

According to a first aspect, the invention relates to an apparatus for steam heating a biomass sludge. According to a second aspect, the invention relates to an apparatus for continuous preparation of a disintegrated biomass sludge using a disintegration device. According to a third aspect, the invention relates to a method for continuously preparing a disintegrated biomass sludge using the apparatus according to the second aspect of the invention. The present invention may be employed for hydrothermal carbonisation (HTC) processes or other hydrothermal or sludge hygienisation processes.

### Background Art

In the past, many efforts have been made to imitate the natural coalification of biomass, which takes place on a time scale of some hundred (peat) to hundred million (black coal) years. Besides the formation of charcoal by pyrolysis of dry biomass, the so-called hydrothermal carbonisation (HTC) process for the manufacture of coal or coal-like materials has recently attracted increasing attention.

While the principle of hydrothermal transformation of cellulose into coal-like materials was developed as early as 1913 by Bergius, the hydrothermal carbonisation has recently seen a renaissance triggered by the reports of M. Antonietti. In 2006, M. Antonietti reported that coal-like material can be obtained by heating biomass and water in the presence of a catalyst in a pressure vessel at 180°C for 12 hours. The research group of M. Antionietti further optimised the HTC process. For instance, M.M.Titirici et al., in New J. Chem., 31 (2007), pp. 787-789 described the catalysed HTC as an attractive alternative for the sequestration of carbon from biomass to treat the CO₂ problem. According to the paper, the optimum reaction conditions involve heating biomass dispersion under weakly acidic conditions in a closed reaction vessel for 4 - 24 h to temperatures of around 200°C.

WO 2008/138637 relates to a process for the preparation of coal or humus from biomass by hydrothermal carbonisation. The process is **characterized in that** the internal temperature of the reactor is controlled by discharging reaction heat from the reactor in the form of steam. The steam can also be used to heat the biomass in a pre-heating unit arranged upstream of the reactor.

However, the known processes for the preparation of coal-like materials by hydrothermal carbonisation as described above left much to be desired in terms of heat management, as well as yield, efficiency and quality control of the final coal-like material.

WO-A-2010/092040 discloses a hydrothermal process for the preparation of coal-like material from biomass and an evaporation column which can be used in this process. The column is equipped with one or more steam injection valves for injecting pressurised steam into a biomass slurry.

GB-A-2 376 461 discloses an apparatus and a method for the heating of a sewage sludge by continuously passing sludge through a thermal reactor, injecting steam into a flow of sludge with a steam injector. In order to ensure an efficient mixing of the steam and the sludge, the steam is injected at a pressure of about 2 bar and the sludge is passed around a continuous loop at a high velocity.

WO-A-02/36506 discloses an apparatus and a method for the heat treatment of a sludge by direct, in-line injection of low pressure steam into the flowing sludge in counterflow to the sludge. A steam generator is used to inject the steam and the rate of steam injection is controlled by a processor.

However, the known apparatuses and processes for steam heating a sludge require high steam pressures and/or complicated injection mechanisms in order to achieve a satisfactory mixing of the steam and the sludge.

A system and a method for treating wastewater are disclosed in WO-A-2010/059208. The system comprises a macerator pump for finely grinding solids suspended in a wastewater slurry to reduce their particle size. WO-A-2010/067302 discloses a waste biomass pre-ciigester for producing a waste biomass slurry from waste biomass. The pre-digester comprises a screw conveyor for macerating the waste biomass.

However, the known apparatuses and processes for preparing disintegrated biomass sludges are limited with regard to the allowable amount of solid material in the sludges. In particular, the preparation of sludges with high solid contents tends to lead to a clogging of conventional disintegration devices.

### Summary of the Invention

Accordingly, it is an object of the present invention to provide an apparatus for the heating of a biomass sludge which enables efficient mixing of the biomass sludge with steam or a steam containing gas in a simple manner. A further object of the present invention is to provide an apparatus for the continuous preparation of a disintegrated biomass sludge which allows for the reliable preparation of biomass sludges within a wide solid content range. Moreover, the invention aims to provide a reliable method for continuously preparing a disintegrated biomass sludge using this apparatus.

These goals are achieved by an apparatus with the technical features of claim 1, an apparatus with the technical features of claim 8 and a method with the technical features of claim 15. Preferred embodiments of the invention follow from the dependent claims.

According to the first aspect, the present invention provides an apparatus for the heating of a biomass sludge or slurry, the apparatus comprising a first positive displacement pump, a second positive displacement pump and a first piping connecting an outlet of the first positive displacement pump to an inlet of the second positive displacement pump. The apparatus further comprises a steam mixing device provided in the first piping between the first positive displacement pump and the second positive displacement pump and a steam source which is in fluid communication with the steam mixing device and configured to supply steam or a steam containing gas to the steam mixing device. The first positive displacement pump and the second positive displacement pump are configured so that, in operation of the apparatus, the flow rate of the first positive displacement pump is smaller than the flow rate of the second positive displacement pump. The steam mixing device is configured to mix the steam or steam containing gas supplied by the steam source with a biomass sludge in the first piping.

The two pumps are positive displacement pumps and thus capable of pumping a biomass sludge or slurry (sludge pumps). The pumps may be suitably chosen for a desired operation temperature range. For example, one or both of the pumps may be a screw pump, a rotary pump, a piston pump, a peristaltic pump, a diaphragm pump or the like.

The first positive displacement pump may, e.g., either have a smaller nominal flow rate than the second positive displacement pump or means to vary its speed of rotation. Alternatively, the second pump may have a variable pumping capacity or a variable rotation speed and be capable of achieving a pumping capacity exceeding that of the first pump.

The above-specified serial arrangement of the two pumps having different flow rates, in operation of the apparatus, results in a reduced pressure in the first piping between the first and the second pump. The amount of pressure reduction can be controlled by appropriately setting the difference in flow rates of the two pumps. The reduced pressure in the first piping leads to the sucking in of steam or a steam containing gas from the steam source through the steam mixing device. In this regard, the pump arrangement acts as a vacuum pump, pumping steam or a steam containing gas into the first piping, wherein the sludge itself provides sealing for the two pumps.

Therefore, no separate injection devices are required for injecting steam or a steam containing gas into the first piping. Further, due to the vacuum suction effect described above, the steam or gas to be injected does not have to be provided at a high pressure. Hence, the apparatus of the invention can be particularly advantageously used with condensing gases such as low pressure steam. When the condensing gases come into direct contact with the sludge in the first piping, the reduced pressure in the piping causes condensation in a highly effective manner. In this way, the suction capacity of the pump arrangement for condensing gases such as low pressure steam is multiplied.

The steam mixing device is not particularly limited, as long as it is configured to mix the steam or steam containing gas supplied by the steam source with a biomass sludge in the first piping. For example, the steam mixing device may be a branch pipe tee, which has the advantage of being a particularly simple structure. Such a branch pipe tee comprises three pipe portions connected in the centre of the tee, two of which are extended along the same direction and the third of which is extended in a direction substantially perpendicular to this direction. The branch pipe tee is arranged so that the portions extending along the same direction are oriented in a substantially vertical direction, i.e., like a capital "T" which is rotated clockwise by about 90°. In this configuration, the apparatus is arranged so that a sludge coming from the outlet of the first pump enters the pipe portion branching sideways, i.e., the third pipe portion extending in a direction substantially perpendicular to the other two pipe portions, and steam from the steam source enters the upper pipe portion. A condensation surface is provided by the sludge near the pipe branching. Gravity is guiding the sludge into the downward pipe portion towards the second pump. Steam or gas bubbles are dragged along by the sludge and condense further down the piping, before reaching the second pump, within the second pump and/or behind the second pump, thereby providing the intended heat transfer to the sludge.

Alternatively, the steam mixing device may, for example, be a free fall contactor. Such a device provides the advantage of increasing the steam/sludge contact area by dispersion of the sludge. Substantially, the free fall contactor is similar in general shape to the branch pipe tee and arranged in the same manner, but has a portion with increased diameter at the centre of the T-shape. Preferably, in this configuration, the sludge from the first pump enters the upper pipe portion from the top and the steam from the steam source enters the pipe portion branching sideways. The sludge entering the free fall contactor breaks away from the sidewall of the device by means of gravity and a sudden increase in diameter. Herein, the term "sudden increase" refers to an increase in diameter over an inclination angle of 70° to 90°, preferably substantially 90°. The sludge is then contacted with the steam entering from the side and the sludge/steam/gas mixture is collected at the bottom of the contactor and transported towards the second pump. Preferably, the lower portion of the free fall contactor has a funnel shape so that the sludge/steam/gas mixture can be efficiently collected and guided into the lower pipe portion. However, the apparatus may also be arranged so that the steam enters the free fall contactor through the upper pipe portion.

Further, for example, also multi-hole-plates, nozzles, such as spray nozzles, or standard gassing equipment, such as gas dispersion hollow shaft stirrers, may be used as the steam mixing device.

Moreover, two or more steam mixing devices may be provided in the first piping between the first pump and the second pump. Further, the apparatus may include two or more steam sources of the same or a different type. In this case, each of the steam sources may be connected to the first piping via a separate steam mixing device. However, it is also possible to connect a single steam source to a plurality of steam mixing devices.

Depending on the steam source, the apparatus of the invention can also be used as a gas scrubber, condensing organic matter along with the water into the sludge stream, thus removing it from the (saturated) gas stream, either substituting further gas pollution removal systems or reducing the load for subsequently installed equipment.

Also the steam source is not particularly limited. As described above, the pump arrangement of the apparatus according to the invention acts as a vacuum pump. Hence, the apparatus may take the role of a vacuum in a vacuum drying process to reduce the drying temperature. The steam released in such a drying process is then mixed with a biomass sludge in the first piping by the steam mixing device. In such a configuration, the apparatus may also be used in combination with conventional vacuum equipment, such as water ring pumps. Further, the steam or steam containing gas may also be provided by flash evaporation as it occurs, e.g., in HTC processes where a superheated coal/water slurry is drawn through a valve, nozzle, pressure lock or similar device from a chemical reactor and cooled down thereby. For example, a coal slurry may be drawn from an HTC reactor with saturated processing conditions of 180°C and 11 bar pressure (absolute). Adiabatic pressure reduction to atmospheric pressure results in evaporation of approximately 15% of the water, thus cooling the coal/water/steam mixture by 80K. Such a configuration is particularly advantageous because the steam thus generated can be directly used for heating the biomass input stream of the HTC process by using the apparatus of the invention.

Moreover, the apparatus of the invention may be used for increasing the effectiveness of the evaporation cooling by the application of a vacuum. For example, if the apparatus is operated at a pressure in the first piping between the two pumps of 700 mbar (absolute), the coal slurry is cooled down to 90°C during flash evaporation and the amount of the available low pressure steam is increased by approximately 12%.

The term "biomass" as used herein is broadly understood as encompassing all kinds of plant and animal material and material derived from the same. According to a preferred embodiment, biomass as meant in the present specification shall not include petroleum or petroleum derived products.

The biomass for use in the present invention may comprise macromolecular compounds, examples of which are lignin and polysaccharides, such as starch, cellulose, and glycogen. As used herein, the term "cellulose" is intended to encompass hemicelluloses commonly also referred to as polyoses.

As will be appreciated, certain kinds of biomass may include both, plant and animal -derived material. As examples, manure (dung), night soil and sewage sludge can be mentioned. While the biomass for use in the present invention is preferably plant biomass, i.e., biomass of or derived from plants, certain contents of animal biomass (i.e. biomass of or derived from animals) may be present therein. For instance, the biomass may contain up to 30 % of animal biomass.

Without limitation, examples of biomass are crop, agricultural food and waste, feed crop residues, wood (such as wood flour, wood waste, scrap wood, sawdust, chips and discards), straw (including rice straw), grass, leaves, chaff, and bagasse. Furthermore, industrial and municipal wastes, including waste paper can be exemplified.

The term "biomass" as used herein preferably also includes monosaccharides such as glucose, ribose, xylose, arabinose, mannose, galactose, fructose, sorbose, fucose and rhamnose, as well as oligosaccharides.

The apparatus of the invention may also comprise a means for controlling the pressure level in the first piping between the two pumps. Especially when a high final temperature of the sludge is desired, such a pressure control is advantageous for an effective operation. In particular, if the pressure between the two pumps is falling below the saturation pressure of water at the current temperature of the sludge, the above-mentioned condensation effect may be impaired. For example, a fine control of the pressure level can be achieved by a feedback controller using the pressure level between the pumps and a desired pressure level as input parameters and controlling the motor speed of the variable speed pump accordingly, e.g., by using a PI or PID algorithm.

Moreover, the apparatus of the invention may comprise a means for preventing sludge from being erroneously transported towards the steam source in a reverse direction, e.g., through a steam piping connecting the steam source to the steam mixing device. Such a means can be constituted by a device for detecting condensed matter which is implemented into the steam piping. This device may be, for example, a level switch. Level switches are widely used for the detection of liquid levels, working on a variety of principles. The apparatus may be configured in different ways so as to take different measures when the presence of condensed matter in the steam piping is detected by the detector. For example, the first pump may be switched off if a sludge flow into the steam piping is detected. Further, a valve, preferably of low pressure loss valve type, such as a ball valve or a butterfly valve, may be inserted into the steam piping. This valve can be inserted before or after the detection device and can be closed upon detection of condensed matter in the steam piping.

In one preferred embodiment, the valve is further coupled to the pressure measurement means in such a way that, upon detection of a sludge overflow, the valve is closed, the pressure between the two pumps is hereupon dropping and, once a sufficient pressure difference over the valve is available, the valve is opened, if necessary repeatedly, for a variable time frame, until the sludge is sucked out of the steam piping again and the detector is not detecting any more sludge in the steam piping.

The same or a different valve in the steam piping may be used for another safety mechanism. Before start-up of the apparatus of the invention, this valve is closed. The first and second pumps are started, building up a vacuum in the first piping between them. Only once the pressure in the first piping is below a preset value, which is preferably below atmospheric pressure, the valve is opened and the apparatus is taking up operation. In this way, problematic states of operation during the start up of the pumps can be prevented.

Preferably, for start up of the apparatus of the invention, the two pumps are started in successive order, starting with the second pump. For shut down of the apparatus, the two pumps are shut down in reverse order, starting with the first pump. Alternatively, a time ramp can be implemented in a frequency converter. Both these measures reliably ensure that, at any given time during operation of the apparatus, the flow rate of the second pump is exceeding the flow rate of the first pump, both during start up and shut down.

In one embodiment, the apparatus of the invention further comprises a second piping connecting an outlet of the second positive displacement pump to an inlet of the first positive displacement pump so as to form a closed loop. In this way, the apparatus can be operated in a circulatory manner, which is particularly advantageous if a specific end temperature of the sludge is intended. Specifically, at least a part of the sludge which has been steam heated by means of the steam heating device and is emitted from the second pump can be directly or indirectly returned into the feed stream of the first pump or into a buffer vessel provided in the second piping and capable of accommodating a part of the biomass sludge. Such a vessel can act as a buffer for the first pump. Subsequently the sludge is conveyed to the steam mixing device by the first pump for another heating cycle.

Preferably, the apparatus of the invention further comprises a third pump and a fourth pump, wherein an outlet of the third pump is connected to the second piping and an inlet of the fourth pump is connected to the second piping upstream of a position where the outlet of the third pump is connected to the second piping. In this configuration, the third pump acts as a feed pump for feeding biomass sludge to be heated into the second piping. The fourth pump acts as a discharge pump for discharging heated biomass sludge from the second piping. In this way, the apparatus of the invention can be operated in a fully continuous manner. In particular, the apparatus may be configured so that only part of the heated sludge, e.g., 20% or less, preferably 10% or less, is discharged through the fourth pump and the remaining part of the heated sludge is kept in the second piping to mix with the unheated sludge fed by the third pump. Also the third and fourth pumps may be positive displacement pumps such as the first and second pumps.

In one preferred embodiment, the apparatus of the invention further comprises a first disintegration device which is provided in the second piping and configured to disintegrate a biomass sludge supplied thereto. Herein the term "disintegrate" refers to any kind of treatment which reduces the sizes of solid materials in the sludge. For example, the disintegration device may be a macerator or a screw conveyor.

The first disintegration device may be provided upstream of a position where the inlet of the fourth pump is connected to the second piping. This arrangement provides the advantage of reliably preventing biomass material which has not been disintegrated by the first disintegration device from being discharged through the fourth pump. Moreover, the first disintegration device may be provided upstream of a position where the outlet of the third pump is connected to the second piping. In this case, the fact that a significant pressure drop arises in commonly used disintegration devices can be advantageously utilised. Specifically, this pressure drop results in a low pressure in the second piping downstream of the disintegration device so that only a low pumping pressure of the third pump is required for efficiently feeding biomass material into the second piping. Moreover, the first disintegration device may also be provided between the first positive displacement pump and the steam mixing device.

The implementation of a disintegration device in the apparatus of the invention provides particularly advantageous synergetic effects. For one thing, the condensation of steam, e.g., low pressure steam, also provides part of the water required for many input materials to reach an intended solid content of the produced sludge. For another thing, the steam heating of the sludge, in particular sludge containing biomass fibers, aids in the mechanical disintegration of the sludge. Therefore, the biomass sludge can be disintegrated in a reliable and effective manner.

The apparatus of this embodiment can be operated in a continuous manner, wherein the apparatus may be configured so that only part of the heated and disintegrated sludge, e.g., 20% or less, preferably 10% or less, is discharged through the fourth pump and the remaining part of the heated and disintegrated sludge is kept in the second piping to mix with the unheated, non-disintegrated biomass fed by the third pump. This operation will be described in further detail below with respect to the apparatus of the second aspect of the invention.

As has been mentioned above, the apparatus of the invention may further comprise a buffer vessel (reservoir) provided in the second piping and capable of accommodating a part of the biomass sludge. The implementation of such a buffer vessel allows for the apparatus to be operated also in a batch-wise manner. In this mode of operation, the buffer vessel is filled with a biomass sludge and the two pumps can be operated until a desired criterion is reached. For example, this criterion could be the sludge temperature or processing time, but also external factors, e.g., originating from the steam source. Afterwards, the vessel content is partially or completely discharged and the apparatus is ready for the next cycle.

Alternatively, such a buffer vessel provides a measure for stabilising a continuous mode of operation. Such a vessel may be open towards the atmosphere, have a form of pressure regulation and/or be connected on the gas side to further processing equipment, e.g., for air purification. This is especially advantageous if the provided steam carries significant amounts of non-condensing gases like CO₂,

In this case, the first disintegration device may be provided in the second piping downstream of the position where the outlet of the third pump is connected to the second piping and upstream of the buffer vessel. In such a configuration, it can be reliably ensured that only biomass material which has been disintegrated by the first disintegration device is fed to the buffer vessel.

Preferably, the steam or steam containing gas supplied by the steam source has a pressure of less than 1.5 bar, more preferably less than 1 bar. Such a low pressure steam can be provided in a simple and cost efficient manner, e.g., by using the steam produced in drying processes. Normally, steam produced in drying processes is directly vented into the atmosphere, condensed for heat recovery to heat water or condensed using a cooling infrastructure like cooling water to be able to dispose of it in liquid form. Such steam is a wet, saturated or only lightly overheated steam at a pressure which is only slightly higher than or even below atmospheric pressure.

This low pressure steam carries a large amount of energy. Its heat of condensation is even higher than that of technically used steam under pressure. For example, 1000 kg of saturated steam at atmospheric pressure feature a heat condensation of 2256 GJ or 627 kWh. Due to its low temperature (30-120°C), this energy is commonly only used for low energy operations like heating of water or frequently not used at all. The apparatus of the invention provides an alternative utilisation of such heat streams.

In some applications, it is necessary to heat a thick sludge, e.g., digester input materials from problematic sources, to elevated temperatures (60-120°C). For example, for the use of food leftovers as digester input the legal requirements in Germany call for a hygienisation at a temperature of at least 90°C for at least one hour. For many of these applications, a certain degree of dilution of the input sludge and possible contamination by the steam is well acceptable. In this case, a direct condensation of such steam is a highly efficient option. Such direct condensation allows for heating without indirect heat transfer areas, which are highly problematic and prone to fouling and clogging effects.

As described above, the reduced pressure in the first piping between the two pumps leads to the sucking in of steam or a steam containing gas from the steam source through the steam mixing device. Due to this vacuum effect, no gas injection at high pressures is required and the apparatus of the invention can be readily and efficiently operated with low pressure steam.

The axial length of a portion of the first piping between the steam mixing device and the second positive displacement pump can be chosen so as to provide steam bubbles with an increased time frame for condensation before reaching the second pump, thus reducing cavitations within the pump. Preferably, the axial length of this piping portion is two meters or more.

According to the second aspect, the present invention provides an apparatus for the continuous preparation of a disintegrated biomass sludge or slurry, the apparatus comprising a first positive displacement pump, a first piping connecting an outlet of the first positive displacement pump to an inlet of the first positive displacement pump so as to form a closed loop, a first disintegration device which is provided in the first piping and configured to disintegrate a biomass sludge supplied thereto, a second pump and a third pump. An outlet of the second pump is connected to the first piping and an inlet of the third pump is connected to the first piping upstream of a position where the outlet of the second pump is connected to the first piping. The second and third pumps may be positive displacement pumps as described above, solid feeders, such as screws, and the like. For example, the disintegration device may be a macerator or a screw conveyor.

This apparatus of the invention can be particularly advantageously used for the continuous preparation of high solid content pumpable biomass sludges. In particular, the second pump can act as a feed pump for feeding biomass sludge to be disintegrated into the first piping. Alternatively, two or more such feed pumps may be present. The third pump can act as a discharge pump for discharging disintegrated biomass sludge from the first piping. In this way, the apparatus of the invention can be operated in a fully continuous manner. Specifically, only part of the disintegrated sludge, e.g., 20% or less, preferably 10% or less, may be discharged through the third pump and the remaining part of the disintegrated sludge may be kept in the first piping to mix with the non-disintegrated biomass fed by the second pump. Subsequently, the resultant mixture of the disintegrated biomass sludge and the biomass sludge fed by the second pump is conveyed to the disintegration device by means of the first positive displacement pump for another disintegration cycle.

Thus, the apparatus of the invention allows for a very high solid content of the resulting sludge because the disintegration device is always fed with a mixture of pre-cut material with only some previously untreated material added by the one or more feed pumps.

In one embodiment, the apparatus according to the second aspect of the invention further comprises a fourth positive displacement pump provided in the first piping downstream of the first positive displacement pump, a first steam mixing device provided in the first piping between the first positive displacement pump and the fourth positive displacement pump and a first steam source which is in fluid communication with the first steam mixing device and configured to supply steam or a steam containing gas to the first steam mixing device. The first positive displacement pump and the fourth positive displacement pump are configured so that, in operation of the apparatus, the flow rate of the first positive displacement pump is smaller than the flow rate of the fourth positive displacement pump. The first steam mixing device is configured to mix the steam or steam containing gas supplied by the first steam source with a biomass sludge in the first piping. The fourth positive displacement pump may be provided upstream of the first disintegration device.

As has been described above, such a configuration provides particularly advantageous synergetic effects. Specifically, the condensation of steam, e.g., low pressure steam, also provides part of the water required for many input materials to reach an intended solid content of the produced sludge. Further, the steam heating of the sludge, in particular sludge containing biomass fibers, by means of the first steam mixing device aids in the mechanical disintegration of the sludge. Therefore, the biomass sludge can be disintegrated in a reliable and effective manner.

The apparatus of this embodiment can be operated in a continuous manner, wherein the apparatus can be configured so that only part of the heated and disintegrated sludge, e.g., 20% or less, preferably 10% or less, is discharged through the third pump and the remaining part of the heated and disintegrated sludge is kept in the first piping to mix with the unheated, non-disintegrated biomass fed by the second pump. The resultant mixture of the heated and disintegrated biomass sludge with the biomass sludge fed by the second pump is conveyed to the first steam mixing device by means of the first positive displacement pump and further to the disintegration device by means of the fourth positive displacement pump for another heating and disintegration cycle.

The apparatus according to the second aspect of the invention may further comprise a buffer vessel (reservoir) provided in the first piping and capable of accommodating a part of the biomass sludge. This vessel can act as a buffer for the first positive displacement pump.

In one embodiment, the apparatus of the invention further comprises a second piping connected to an outlet of the third pump and a second disintegration device which is provided in the second piping downstream of the third pump and configured to disintegrate a biomass sludge supplied thereto. The second disintegration device may be of the same type as the first disintegration device or of a different type.

This configuration of the apparatus allows for a second disintegration step to be carried out after the discharge pump. Preferably, the second disintegration device is configured so that the resulting length of biomass fibers in the biomass sludge is smaller than that within the first piping of the apparatus. Accordingly, the second disintegration device may be fed with a flow rate which is smaller than that of the sludge in the first piping. By using this apparatus configuration, the pumpability of the sludge can be significantly enhanced.

In one embodiment, the apparatus of the invention further comprises a fifth pump, wherein an inlet of the fifth pump is connected to the second piping, a second steam mixing device provided in the second piping between the third pump and the fifth pump, and a second steam source which is in fluid communication with the second steam mixing device and configured to supply steam or a steam containing gas to the second steam mixing device. The third pump and the fifth pump are configured so that, in operation of the apparatus, the flow rate of the third pump is smaller than the flow rate of the fifth pump. The second steam mixing device is configured to mix the steam or steam containing gas supplied by the second steam source with a biomass sludge in the second piping. In this case, separate steam sources can be used for the first and second steam sources. However, alternatively, a single steam source may be employed as both the first and the second steam source. The fifth pump may be a positive displacement pump as described above.

In this way, a further steam heating step can be carried out after the discharge pump. The apparatus may be configured so that the arrangement of the third and fifth pumps is operated at a different, preferably higher, pressure than the arrangement of the first and fourth positive displacement pumps. The fifth pump may be part of a processing further down the line, e.g., the high pressure feeding pump of a hydrothermal process. In this case, the third pump can take the role of a booster pump, e.g., rising the pressure on the lower pressure side of the high pressure pump.

Specifically, the third pump and the fifth pump may be configured so that the third pump is operating as a booster pump for the fifth pump, maintaining a pressure level in the second piping that is above that in the first piping and the second steam mixing device may be configured to mix the steam or steam containing gas supplied by the second steam source, which is providing steam at a pressure level above that maintained in the second piping by the third pump and the fifth pump, with a biomass sludge in the second piping. A mechanical disintegration unit proves advantageous for this approach as well. In this case, a heating by low pressure direct condensation after the third pump is not possible. However, direct condensation of steam from other sources can be utilised, e.g., with steam spargers. In this mode of operation, sludge temperatures above 100°C are achievable.

For example, in an HTC facility, the circulatory system is working with low pressure steam from the flash evaporation of the coal slurry and is heating the biomass input stream to 80°C while operating at 800 mbar absolute pressure. The discharge pump, i.e., the third pump, is feeding this material through a sparger and a second disintegration device into the high pressure feeding pump of the HTC process, i.e., the fifth pump. The pressure after the discharge pump is controlled and set to a pressure of 2.5 bar absolute. The sparger is operated with steam drawn from the HTC process with a pressure of 7-12 bar absolute. A valve in the steam line is limiting the temperature of the biomass to the maximum operational temperature of the high pressure feeding pump, e.g., 115°C.

In one embodiment, the apparatus of the invention comprises a means for measuring the total mass of sludge within the circulatory part of the apparatus, e.g., by a liquid level measurement within the buffer vessel, preferably using a contact-free principle like ultrasonic or radar or simply visual inspection. The buffer vessel may be refilled, preferably with material with a solid content exceeding that of the desired pumpable sludge, by appropriate equipment, e.g., feeding screws or sludge pumps. This refilling can be handled manually or by a closed control loop.

Moreover, the apparatus of the invention may comprise a means for measuring the temperature within the apparatus at an arbitrary point within the loop, preferably after the fourth positive displacement pump. If a limitation of the temperature is required for any reason, a control loop either using a continuous control valve or a shut-off valve can be implemented.

Moreover, the apparatus of the invention may comprise a means for controlling the fraction of solids in the biomass sludge in the first piping or within the entire apparatus. Such a configuration is particularly advantageous if material of significantly higher solid content than the intended pumpable sludge is treated within the apparatus. The measurement of the total amount of solids can be implemented either by direct methods, e.g., by measuring the water content by capacity sensors, or preferably indirectly by monitoring pressure differences or preferably electrical power uptakes by one or several pumps within the apparatus. Solid contents exceeding the intended range of values are then compensated by the addition of water and/or water based waste streams with a solid content below that of the intended pumpable sludge.

For example, one ton per hour of leaves with a solid content of 40% having a temperature of 15°C are to be turned into a pumpable sludge with short fiber length, a solid content of 25% and a temperature of 90°C. A steam source with 90°C saturated steam and a water source at 70°C are available. The apparatus using a controlling scheme as described above would produce a mixture of approximately 1000 kg/h leaves, 480 kg/h hot water and 120 kg/h of low pressure steam being processed and resulting in 1600 kg/h of the desired product sludge.

Preferably, the steam or steam containing gas supplied by the first and/or the second steam source has a pressure of less than 1.5 bar, more preferably less than 1 bar. In this way, the advantageous effects already described in detail above can be achieved.

According to the third aspect, the invention provides a method for continuously preparing a disintegrated biomass sludge or slurry using the apparatus according to the second aspect of the invention. The method comprises the steps of continuously feeding a biomass sludge to the first piping by means of the second pump, conveying the biomass sludge fed by the second pump to the first disintegration device by means of at least the first positive displacement pump and disintegrating the biomass sludge conveyed by at least the first positive displacement pump by means of the first disintegration device so as to produce a disintegrated biomass sludge. Further, the method comprises the steps of continuously discharging a part, e.g., 20% or less, preferably 10% or less, of the disintegrated biomass sludge by means of the third pump, conveying the remaining part of the disintegrated biomass sludge towards the disintegration device by means of at least the first positive displacement pump, so that the disintegrated biomass sludge mixes with the biomass sludge fed by the second pump, and conveying the resultant mixture of the disintegrated biomass sludge and the biomass sludge fed by the second pump to the disintegration device by means of at least the first positive displacement pump.

This method provides the advantageous effects already described above. In particular, the method can be particularly advantageously used for the continuous preparation of high solid content pumpable biomass sludges.

The method of the invention allows for a very high solid content of the resulting sludge because the disintegration device is always fed with a mixture of pre-cut material with only some previously untreated material added by the one or more feed pumps.

Preferably, the method of the invention further comprises the steps of measuring and controlling the total mass of the biomass sludge in the first piping, the temperature of the biomass sludge in the first piping and/or the fraction of solids in the biomass sludge in the first piping. These steps can be carried out, for example, in the ways already described in detail above.

### Brief Description of the Drawings

Hereinafter, non-limiting examples are explained with reference to the drawings, in which:
Figure 1 shows a schematic view of an apparatus for the heating of a biomass sludge or slurry according to an embodiment of the present invention;
Figure 2 shows a schematic view of an apparatus for the heating of a biomass sludge or slurry according to another embodiment of the present invention;
Figure 3 shows a schematic view of a steam mixing device according to an embodiment of the present invention;
Figure 4 shows a schematic view of a steam mixing device according to another embodiment of the present invention; and
Figure 5 shows a schematic view of an apparatus for the heating and disintegrating of a biomass sludge or slurry according to an embodiment of the present invention.

### Detailed Description of Preferred Embodiments

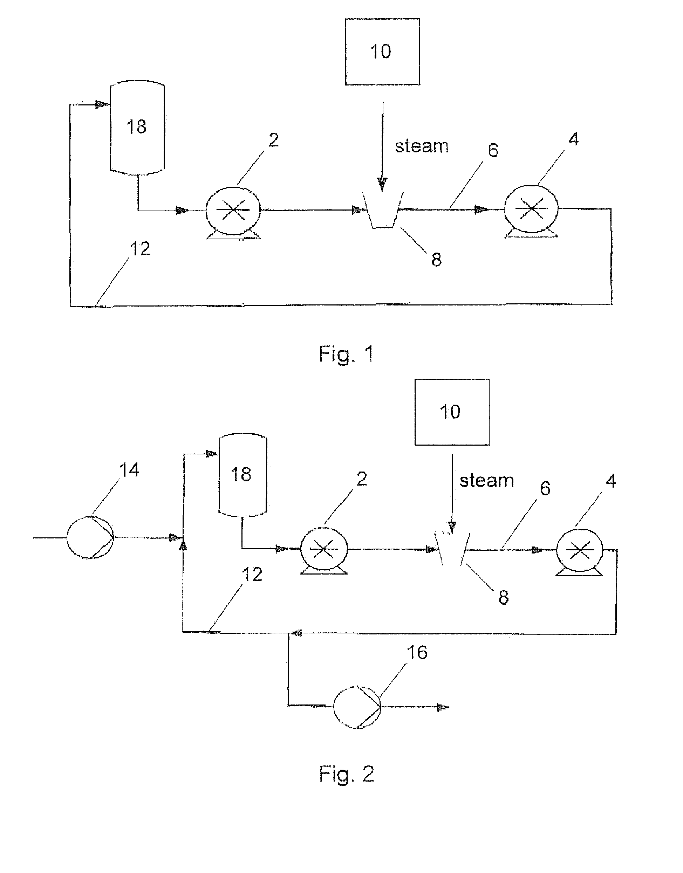

Figure 1 shows a schematic view of an apparatus for the heating of a biomass sludge or slurry according to a preferred embodiment of the present invention.

The apparatus shown in Fig. 1 comprises a first positive displacement pump 2, a second positive displacement pump 4 and a first piping 6 connecting an outlet of the first positive displacement pump 2 to an inlet of the second positive displacement pump 4. The apparatus further comprises a steam mixing device 8 provided in the first piping 6 between the first positive displacement pump 2 and the second positive displacement pump 4 and a steam source 10 which is in fluid communication with the steam mixing device 8 and configured to supply steam or a steam containing gas to the steam mixing device 8. The first positive displacement pump 2 and the second positive displacement pump 4 are configured so that, in operation of the apparatus, the flow rate of the first positive displacement pump 2 is smaller than the flow rate of the second positive displacement pump 4. The steam mixing device 8 is configured to mix the steam or steam containing gas supplied by the steam source 10 with a biomass sludge in the first piping 6. The steam source 10 may, for example, provide steam or a steam containing gas generated in a vacuum drying process or by flash evaporation.

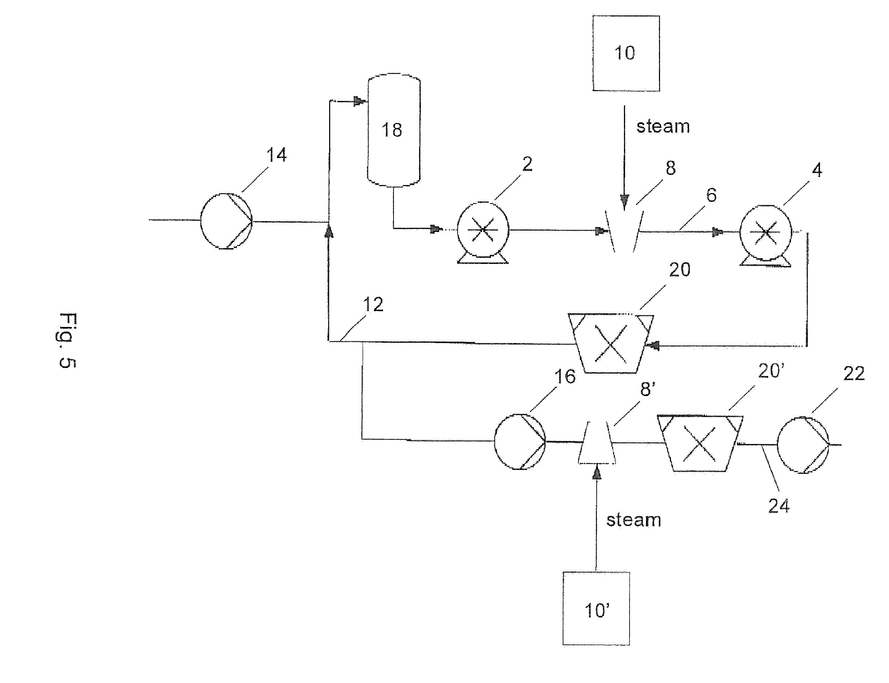

Further, the apparatus comprises a second piping 12 connecting an outlet of the second positive displacement pump 4 to an inlet of the first positive displacement pump 2 so as to form a closed loop. The arrows in the first and second pipings 6, 12 of Fig. 1 indicate a flow direction of the biomass sludge in the apparatus (the same applies to Figs. 2 and 5). Moreover, a buffer vessel (reservoir) 18 is provided in the second piping 12 which is capable of accommodating a part of the biomass sludge. Hence, the apparatus may be operated in a circulatory or a batch-wise mode, as has been detailed above.

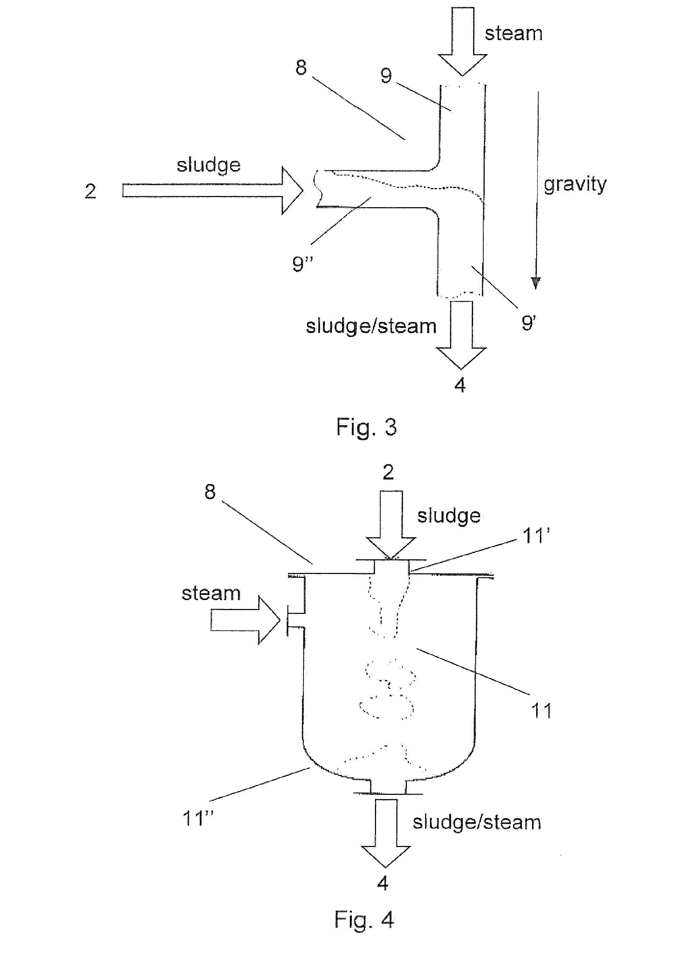

For example, the two pumps 2, 4 may be rotary lobe pumps each having a nominal pump capacity of 35 m³/h. In this case, the required difference in flow rates may be achieved by a frequency converter for controlling the speed of rotation of the first pump 2. For such a configuration of the apparatus shown in Fig. 1, an absolute pressure of approximately 200 mbar in the first piping 6 between the pumps 2, 4 can be obtained by operating the second pump 4 at 50 Hz and reducing the speed of rotation of the first pump 2 by setting the motor frequency to 45 Hz. This low pressure in the first piping 6 leads to the sucking in of steam or a steam containing gas from the steam source 10 into the first piping 6 without any sludge being evicted from the first piping 6. The sucked in steam or steam containing gas is mixed with a biomass sludge in the first piping 6, as will be described in detail below with reference to Figs. 3 and 4.

Figure 2 shows a schematic of an apparatus for the heating of a biomass sludge or slurry according to another preferred embodiment of the present invention. The configuration of the apparatus of Fig. 2 is substantially identical to that of the apparatus shown in Fig. 1, as is indicated by the use of the same reference signs. However, the apparatus of Fig. 2 further comprises a third pump 14 and a fourth pump 16. An outlet of the third pump 14 is connected to the second piping 12 and an inlet of the fourth pump 16 is connected to the second piping 12 upstream of a position where the outlet of the third pump 14 is connected to the second piping 12.

In this configuration, the third pump 14 acts as a feed pump for feeding biomass sludge to be heated into the second piping 12. The fourth pump 16 acts as a discharge pump for discharging heated biomass sludge from the second piping 12. In this way, the apparatus shown in Fig. 2 can be operated in a fully continuous manner. In particular, the apparatus may be configured so that only part of the sludge heated by the steam mixing device 8 is discharged through the fourth pump 16 and the remaining part of the heated sludge is kept in the second piping 12 to mix with the unheated sludge fed by the third pump 14. Also the third and fourth pumps 14, 16 may be positive displacement pumps such as the first and second pumps 2, 4, e.g., rotary lobe pumps.

In the following, two preferred embodiments of the steam mixing device 8 which may be used in the apparatuses shown in Figs. 1 and 2 will be described in detail with reference to Figs. 3 and 4.

Figure 3 shows an embodiment in which a branch pipe tee is used as the steam mixing device 8. As can be seen from Fig. 3, the branch pipe tee comprises three pipe portions 9, 9', 9" connected in the centre of the tee, two of which 9, 9' are extended along the same direction and the third of which 9" is extended in a direction substantially perpendicular to this direction. The branch pipe tee is arranged so that the portions extending along the same direction 9, 9' are oriented in a substantially vertical direction, i.e., like a capital "T" which is rotated clockwise by about 90°. In this configuration, the apparatus is configured so that a sludge coming from the outlet of the first pump 2 enters the pipe portion 9" branching sideways, i.e., the third pipe portion 9" extending in a direction substantially perpendicular to the other two pipe portions 9, 9' and steam from the steam source 10 enters the upper pipe portion 9, as is indicated by respective arrows in Fig. 3.

A condensation surface, which is designated by a dotted line in Fig. 3, is provided by the sludge near the pipe branching. Gravity is guiding the sludge into the downward pipe portion 9' towards the second pump 4, as is indicated by an arrow in Fig. 3. Steam or gas bubbles are dragged along by the sludge and condense further down the piping, before reaching the second pump 4, within the second pump 4 and/or behind the second pump 4, thereby providing the intended heat transfer to the sludge. Such a structure of the steam mixing device 8, for example, has the advantage that it is particularly simple.

Figure 4 shows an embodiment in which a free fall contactor is used as the steam mixing device 8. Such a device provides the advantage of increasing the steam/sludge contact area by dispersion of the sludge. The direction of gravity in the arrangement of Fig. 4 is the same as that indicated by an arrow in Fig. 3.

As can be seen from Fig. 4, the free fall contactor is similar in general shape to the branch pipe tee and arranged in the same manner, but has a portion 11 with increased diameter at the centre of the T-shape. In the configuration shown in Fig. 4, the sludge from the first pump 2 enters the upper pipe portion from the top and the steam from the steam source 10 enters the pipe portion branching sideways, as is indicated by respective arrows in Fig. 4. The sludge entering the free fall contactor breaks away from the sidewall of the device by means of gravity and a sudden increase in diameter at the upper portion 11' thereof. The dispersed sludge is indicated by the dotted lines in Fig. 4.

As can be seen in Fig. 4, the increase in diameter occurs over an inclination angle of substantially 90°. The sludge is then contacted with the steam entering from the side and the sludge/steam/gas mixture is subsequently collected at the bottom of the contactor and transported towards the second pump 4. The lower portion 11" of the free fall contactor has a funnel shape so that the sludge/steam/gas mixture can be efficiently collected and guided into the lower pipe portion.

Figure 5 shows a schematic view of an apparatus for the heating and disintegrating of a biomass sludge or slurry according to a preferred embodiment of the present invention.

The apparatus shown in Fig. 5 represents a combination of the apparatuses of the first and second aspects of the present invention. Herein, the first pump 2, the second pump 4, the third pump 14 and the fourth pump 16 of the apparatus according to the first aspect of the invention correspond to the first pump 2, the fourth pump 4, the second pump 14 and the third pump 16 of the apparatus according to the second aspect of the invention, respectively. The first piping 6, 12 of the apparatus according to the second aspect of the invention corresponds to a combination of the first 6 and second 12 pipings of the apparatus according to the first aspect of the invention. In the following description of Fig. 5, the terminology as used for the second aspect of the invention will be employed.

The general structure of the apparatus shown in Fig. 5 is similar to that of the apparatus shown in Fig. 2, as is indicated by the use of the same reference signs. However, the apparatus of Fig. 5 further comprises a fifth pump 22, a second piping 24 connecting an outlet of the third pump 16 to an inlet of the fifth pump 22, a first disintegration device 20 which is provided in the first piping 12 upstream of a position where the inlet of the third pump 16 is connected to the first piping 12 and which is configured to disintegrate a biomass sludge supplied thereto, and a second disintegration device 20' which is provided in the second piping 24 between the third pump 16 and the fifth pump 22 and configured to disintegrate a biomass sludge supplied thereto.

Moreover, the apparatus of Fig. 5 further comprises a second steam mixing device 8' provided in the second piping 24 between the third pump 16 and the fifth pump 22 and a second steam source 10' which is in fluid communication with the second steam mixing device 8' and configured to supply steam or a steam containing gas to the second steam mixing device 8'. The third pump 16 and the fifth pump 22 are configured so that, in operation of the apparatus, the flow rate of the third pump 16 is smaller than the flow rate of the fifth pump 22. The second steam mixing device 8' is configured to mix the steam or steam containing gas supplied by the second steam source 10' with a biomass sludge in the second piping 24.

As has been described in detail above, such a combined configuration provides particularly advantageous synergetic effects. Specifically, the condensation of steam, e.g., low pressure steam, also provides part of the water required for many input materials to reach an intended solid content of the produced sludge.

The second steam mixing device 8' may be provided in the second piping 24 between the third pump 16 and the second disintegration device 20', as is shown in Fig. 5. In this case, the steam heating of the sludge, in particular sludge containing biomass fibers, by means of the second steam mixing device 8' aids in the mechanical disintegration of the sludge by the second disintegration device 20'. Therefore, the biomass sludge can be disintegrated in a reliable and effective manner.

Alternatively, the second steam mixing device 8' may be provided in the second piping 24 between the second disintegration device 20' and the fifth pump 22. Such an arrangement provides the advantage that also a disintegration device having a relatively low operating temperature may be used as the second disintegration device 20'.

Moreover, the implementation of a second disintegration device 20' allows for a second disintegration step to be carried out after the third pump 16. The second disintegration device 20' may be configured so that the resulting length of biomass fibers in the biomass sludge is smaller than that within the first piping 6, 12 of the apparatus. Accordingly, the second disintegration device 20' may be fed with a flow rate which is smaller than that of the sludge in the first piping 6, 12. By using this apparatus configuration, the pumpability of the sludge can be significantly enhanced.

For example, the first disintegration device 20 may be a macerator with a cutting space of 22 mm operating at a rate of 10 m³/h and the second disintegration device 20' may be a macerator with a cutting space of 8 mm operating at a rate of 1 m³/h, When such an apparatus was operated with a corn based sludge having solid contents between 24 and 26%, the observed fiber length in samples taken from the circulatory system went up to 35 mm, whereas in the discharged material only very few fibers longer than 10 mm were found.

The apparatus of this embodiment can be operated in a continuous manner, wherein only part, e.g., 20% or less, preferably 10% or less, of the heated and disintegrated sludge is discharged through the third pump 16 and the remaining part of the heated and disintegrated sludge is kept in the first piping 6, 12 to mix with the unheated, non-disintegrated biomass fed by the second pump 14. The resultant mixture of the heated and disintegrated biomass sludge with the biomass sludge fed by the second pump 14 is conveyed to the first steam mixing device 8 and further to the disintegration device 20 by means of the first positive displacement pump 2 for another heating and disintegration cycle.

This circulatory mixing is particularly advantageous for the preparation of biomass sludges with high solid contents. For example, operating the apparatus shown in Fig. 5 with a mixture of water and corn silage, which is highly fibrous material tending to clog disintegration devices if fed directly without circulatory mixing, resulted in well pumpable sludge with a total solid content of 26.4%, even without the addition of steam.

Moreover, due to the implementation of a second steam mixing device 8' and a second steam source 10', a further steam heating step can be carried out after the third pump 16. In this way, a particularly homogeneous heating of the biomass sludge can be achieved.

Further, the apparatus shown in Fig. 5 may be configured so that the arrangement of the third and fifth pumps 16, 22 is operated at a higher pressure than the arrangement of the first and fourth positive displacement pumps 2, 4. Also, the fifth pump 22 may be part of a processing further down the line, e.g., the high pressure feeding pump of a hydrothermal process. In this case, the third pump 16 can take the role of a booster pump, e.g., rising the pressure on the lower pressure side of the high pressure pump.

The following numbered paragraphs provide further disclosure of the invention:
1. An apparatus for the heating of a biomass sludge, the apparatus comprising:
   a first positive displacement pump (2),
   a second positive displacement pump (4),
   a first piping (6) connecting an outlet of the first positive displacement pump (2) to an inlet of the second positive displacement pump (4),
   a steam mixing device (8) provided in the first piping (6) between the first positive displacement pump (2) and the second positive displacement pump (4), and
   a steam source (10) which is in fluid communication with the steam mixing device (8) and configured to supply steam or a steam containing gas to the steam mixing device (8), wherein
   the first positive displacement pump (2) and the second positive displacement pump (4) are configured so that, in operation of the apparatus, the flow rate of the first positive displacement pump (2) is smaller than the flow rate of the second positive displacement pump (4), and
   the steam mixing device (8) is configured to mix the steam or steam containing gas supplied by the steam source (10) with a biomass sludge in the first piping (6).
2. The apparatus according to paragraph 1, further comprising a second piping (12) connecting an outlet of the second positive displacement pump (4) to an inlet of the first positive displacement pump (2) so as to form a closed loop.
3. The apparatus according to paragraph 2, further comprising a third pump (14) and a fourth pump (16), wherein an outlet of the third pump (14) is connected to the second piping (12) and an inlet of the fourth pump (16) is connected to the second piping (12) upstream of a position where the outlet of the third pump (14) is connected to the second piping (12).
4. The apparatus according to paragraph 3, further comprising a first disintegration device (20) which is provided in the second piping (12) and configured to disintegrate a biomass sludge supplied thereto.
5. The apparatus according to any one of paragraphs 2 to 4, further comprising a buffer vessel (18) provided in the second piping (12) and capable of accommodating a part of the biomass sludge.
6. The apparatus according to any one of the preceding paragraphs, wherein the steam or steam containing gas has a pressure of less than 1.5 bar, preferably less than 1 bar.
7. The apparatus according to any one of the preceding paragraphs, wherein an axial length of a portion of the first piping (6) between the steam mixing device (8) and the second positive displacement pump (4) is two meters or more.
8. An apparatus for the continuous preparation of a disintegrated biomass sludge, the apparatus comprising:
   a first positive displacement pump (2),
   a first piping (6, 12) connecting an outlet of the first positive displacement pump (2) to an inlet of the first positive displacement pump (2) so as to form a closed loop,
   a first disintegration device (20) which is provided in the first piping (6, 12) and configured to disintegrate a biomass sludge supplied thereto,
   a second pump (14), and
   a third pump (16), wherein
   an outlet of the second pump (14) is connected to the first piping (6, 12) and an inlet of the third pump (16) is connected to the first piping (6, 12) upstream of a position where the outlet of the second pump (14) is connected to the first piping (6, 12).
9. The apparatus according to paragraph 8, further comprising:
   a fourth positive displacement pump (4) provided in the first piping (6, 12) downstream of the first positive displacement pump (2),
   a first steam mixing device (8) provided in the first piping (6, 12) between the first positive displacement pump (2) and the fourth positive displacement pump (4), and
   a first steam source (10) which is in fluid communication with the first steam mixing device (8) and configured to supply steam or a steam containing gas to the first steam mixing device (8), wherein
   the first positive displacement pump (2) and the fourth positive displacement pump (4) are configured so that, in operation of the apparatus, the flow rate of the first positive displacement pump (2) is smaller than the flow rate of the fourth positive displacement pump (4), and
   the first steam mixing device (8) is configured to mix the steam or steam containing gas supplied by the first steam source (10) with a biomass sludge in the first piping (6, 12).
10. The apparatus according to paragraph 8 or 9, further comprising:
   a second piping (24) connected to an outlet of the third pump (16) and
   a second disintegration device (20') which is provided in the second piping (24) downstream of the third pump (16) and configured to disintegrate a biomass sludge supplied thereto.
11. The apparatus according to paragraph 10, further comprising:
   a fifth pump (22), wherein an inlet of the fifth pump (22) is connected to the second piping (24),
   a second steam mixing device (8') provided in the second piping (24) between the third pump (16) and the fifth pump (22), and
   a second steam source (10') which is in fluid communication with the second steam mixing device (8') and configured to supply steam or a steam containing gas to the second steam mixing device (8'), wherein
   the third pump (16) and the fifth pump (22) are configured so that, in operation of the apparatus, the flow rate of the third pump (16) is smaller than the flow rate of the fifth pump (22), and
   the second steam mixing device (8') is configured to mix the steam or steam containing gas supplied by the second steam source (10') with a biomass sludge in the second piping (24).
12. The apparatus according to paragraph 10, further comprising
   a fifth pump (22), wherein an inlet of the fifth pump (22) is connected to the second piping (24),
   a second steam mixing device (8') provided in the second piping (24) between the third pump (16) and the fifth pump (22), and
   a second steam source (10') which is in fluid communication with the second steam mixing device (8') and configured to supply steam or a steam containing gas to the second steam mixing device (8'), wherein
   the third pump (16) and the fifth pump (22) are configured so that the third pump (16) is operating as a booster pump for the fifth pump (22), maintaining a pressure level in the second piping (24) that is above that in the first piping (6,12), and
   the second steam mixing device (8') is configured to mix the steam or steam containing gas supplied by the second steam source (10'), which is providing steam at a pressure level above that maintained in the second piping (24) by the third pump (16) and the fifth pump (22), with a biomass sludge in the second piping (24).
13. The apparatus according to any one of paragraphs 8 to 12, further comprising a buffer vessel (18) provided in the first piping (6, 12) and capable of accommodating a part of the biomass sludge.
14. The apparatus according to any one of paragraphs 9 to 13, wherein the steam or steam containing gas supplied by the first (10) and/or the second steam source (10') has a pressure of less than 1.5 bar, preferably less than 1 bar.
15. A method for continuously preparing a disintegrated biomass sludge using the apparatus according to any one of paragraphs 8 to 14, the method comprising the steps of:
   continuously feeding a biomass sludge to the first piping (6, 12) by means of the second pump (14),
   conveying the biomass sludge fed by the second pump (14) to the first disintegration device (20) by means of at least the first positive displacement pump (2),
   disintegrating the biomass sludge conveyed by at least the first positive displacement pump (2) by means of the first disintegration device (20) so as to produce a disintegrated biomass sludge,
   continuously discharging a part of the disintegrated biomass sludge by means of the third pump (16),
   conveying the remaining part of the disintegrated biomass sludge towards the disintegration device (20) by means of at least the first positive displacement pump (2), so that the disintegrated biomass sludge mixes with the biomass sludge fed by the second pump (14), and
   conveying the resultant mixture of the disintegrated biomass sludge and the biomass sludge fed by the second pump (14) to the disintegration device (20) by means of at least the first positive displacement pump (2).
16. The method according to paragraph 15, further comprising the steps of measuring and controlling the total mass of the biomass sludge in the first piping (6, 12), the temperature of the biomass sludge in the first piping (6, 12) and/or the fraction of solids in the biomass sludge in the first piping (6, 12).

## Claims

1. An apparatus for the continuous preparation of a disintegrated biomass sludge, the apparatus comprising:
a first positive displacement pump (2),
a first piping (6, 12) connecting an outlet of the first positive displacement pump (2) to an inlet of the first positive displacement pump (2) so as to form a closed loop,
a first disintegration device (20) which is provided in the first piping (6, 12) and configured to disintegrate a biomass sludge supplied thereto,
a second pump (14), and
a third pump (16), wherein
an outlet of the second pump (14) is connected to the first piping (6, 12) and an inlet of the third pump (16) is connected to the first piping (6, 12) upstream of a position where the outlet of the second pump (14) is connected to the first piping (6, 12).

2. The apparatus according to claim 1, further comprising:
a fourth positive displacement pump (4) provided in the first piping (6, 12) downstream of the first positive displacement pump (2),
a first steam mixing device (8) provided in the first piping (6, 12) between the first positive displacement pump (2) and the fourth positive displacement pump (4), and
a first steam source (10) which is in fluid communication with the first steam mixing device (8) and configured to supply steam or a steam containing gas to the first steam mixing device (8), wherein
the first positive displacement pump (2) and the fourth positive displacement pump (4) are configured so that, in operation of the apparatus, the flow rate of the first positive displacement pump (2) is smaller than the flow rate of the fourth positive displacement pump (4), and
the first steam mixing device (8) is configured to mix the steam or steam containing gas supplied by the first steam source (10) with a biomass sludge in the first piping (6, 12).

3. The apparatus according to claim 1 or 2, further comprising:
a second piping (24) connected to an outlet of the third pump (16) and
a second disintegration device (20') which is provided in the second piping (24) downstream of the third pump (16) and configured to disintegrate a biomass sludge supplied thereto.

4. The apparatus according to claim 3, further comprising:
a fifth pump (22), wherein an inlet of the fifth pump (22) is connected to the second piping (24),
a second steam mixing device (8') provided in the second piping (24) between the third pump (16) and the fifth pump (22), and
a second steam source (10') which is in fluid communication with the second steam mixing device (8') and configured to supply steam or a steam containing gas to the second steam mixing device (8'), wherein
the third pump (16) and the fifth pump (22) are configured so that, in operation of the apparatus, the flow rate of the third pump (16) is smaller than the flow rate of the fifth pump (22), and
the second steam mixing device (8') is configured to mix the steam or steam containing gas supplied by the second steam source (10') with a biomass sludge in the second piping (24).

5. The apparatus according to claim 3, further comprising
a fifth pump (22), wherein an inlet of the fifth pump (22) is connected to the second piping (24),
a second steam mixing device (8') provided in the second piping (24) between the third pump (16) and the fifth pump (22), and
a second steam source (10') which is in fluid communication with the second steam mixing device (8') and configured to supply steam or a steam containing gas to the second steam mixing device (8'), wherein
the third pump (16) and the fifth pump (22) are configured so that the third pump (16) is operating as a booster pump for the fifth pump (22), maintaining a pressure level in the second piping (24) that is above that in the first piping (6,12), and
the second steam mixing device (8') is configured to mix the steam or steam containing gas supplied by the second steam source (10'), which is providing steam at a pressure level above that maintained in the second piping (24) by the third pump (16) and the fifth pump (22), with a biomass sludge in the second piping (24).

6. The apparatus according to any one of claims 1 to 5, further comprising a buffer vessel (18) provided in the first piping (6, 12) and capable of accommodating a part of the biomass sludges.

7. The apparatus according to any one of claims 2 to 6, wherein the steam or steam containing gas supplied by the first (10) and/or the second steam source (10') has a pressure of less than 1.5 bar, preferably less than 1 bar.

8. A method for continuously preparing a disintegrated biomass sludge using the apparatus according to any one of claims 1 to 7, the method comprising the steps of:
continuously feeding a biomass sludge to the first piping (6, 12) by means of the second pump (14),
conveying the biomass sludge fed by the second pump (14) to the first disintegration device (20) by means of at least the first positive displacement pump (2),
disintegrating the biomass sludge conveyed by at least the first positive displacement pump (2) by means of the first disintegration device (20) so as to produce a disintegrated biomass sludge,
continuously discharging a part of the disintegrated biomass sludge by means of the third pump (16),
conveying the remaining part of the disintegrated biomass sludge towards the disintegration device (20) by means of at least the first positive displacement pump (2), so that the disintegrated biomass sludge mixes with the biomass sludge fed by the second pump (14), and
conveying the resultant mixture of the disintegrated biomass sludge and the biomass sludge fed by the second pump (14) to the disintegration device (20) by means of at least the first positive displacement pump (2).

9. The method according to claim 8, further comprising the steps of measuring and controlling the total mass of the biomass sludge in the first piping (6, 12), the temperature of the biomass sludge in the first piping (6, 12) and/or the fraction of solids in the biomass sludge in the first piping (6, 12).
